# EUROPÄISCHE PATENTANMELDUNG

(11) **EP 1 120 154 A2**
(43) Veröffentlichungstag der Anmeldung: **01.08.2001**
(21) Anmeldenummer: 00124580.2
(22) Anmeldetag: 10.11.2000
(51) Int. Cl.: B01J 8/02, B01J 8/08

(54) **Verfahren zur Durchführung homogen katalysierter Umsetzungen**

(30) Priorität: 27.01.2000 DE 10003317
(71) Anmelder: Degussa AG, 40474 Düsseldorf (DE)
(72) Erfinder: Haas, Thomas, Dr., 60316 Frankfurt am Main (DE)

(57) **Zusammenfassung**

Die Umsatzrate einer homogen katalysierten Umsetzung, wobei ein Edukt unter Abspaltung oder Anlagerung eines Reaktionspartners oder unter Isomerisierung in flüssiger Phase, welche einen für die Umsetzung geeigneten Katalysator in wirksamer Menge in gelöster Form enthält, in ein Reaktionsprodukt überführt wird, lässt sich wesentlich steigern, indem die das Edukt und den homogenen Katalysator enthaltende flüssige Phase sowie, soweit erforderlich, anzulagernde Reaktionspartner mit einem stationären oder bewegten Bett aus porösen Partikeln, insbesondere solchen mit einem Porenvolumen von 0,1 bis 3 ml/g, in Kontakt gebracht wird. Bevorzugt wird die flüssige Phase in Rieselbett-Fahrweise über ein stationäres Festbett geleitet.

## Beschreibung

Die Erfindung betrifft ein Verfahren zur Durchführung homogen katalysierter Umsetzungen, wobei ein Edukt unter Anlagerung oder Abspaltung eines Reaktionspartners oder unter Isomerisierung in flüssiger Phase, welche einen für die gewünschte Umsetzung geeigneten gelösten Katalysator in wirksamer Menge enthält, in ein Reaktionsprodukt überführt wird. Durch das erfindungsgemäße Verfahren wird die Umsetzung weiter beschleunigt.

Zahlreiche chemische Umsetzungen werden zwecks Beschleunigung derselben in Gegenwart eines homogenen oder heterogenen Katalysators durchgeführt. Homogene Katalysatoren sind solche, welche im Reaktionsmedium molekulardispers verteilt sind, beispielsweise in einem flüssigen Reaktionsmedium gelöst sind. Heterogene Katalysatoren sind in einem flüssigen Reaktionsmedium unlöslich und umfassen feste Oberflächen und partikelförmige Stoffe, welche die eigentlichen katalytisch wirksamen Zentren aufweisen. Sofern nicht im wesentlichen gleichzeitig oder unmittelbar hintereinander zwei unterschiedliche Reaktionen in einem flüssigen Reaktionsmedium durchgeführt werden sollen, entscheidet sich der Fachmann für die Verwendung eines einzigen, also eines homogenen oder heterogenen Katalysatorsystems. Zur Durchführung beispielsweise einer säurekatalysierten Umsetzung in flüssiger Phase enthält demgemäß das Reaktionsmedium entweder eine gelöste Säure als Homogenkatalysator oder alternativ hierzu wird die Umsetzung unter Verwendung eines saure Aktivitätszentren enthaltenden Heterogenkatalysators durchgeführt.

Das heute üblicherweise angewandte Konzept zur Beschreibung der Wirkungsweise eines Katalysators ist die Annahme, dass am Katalysator ein Zentrum vorliegt, das mit dem Reaktanden einen Komplex bildet und von dem nach der Reaktion die Produkte desorbieren, wobei das ursprüngliche Zentrum wiederhergestellt wird (siehe "Heterogeneous Catalysis in Industrial practice", Charles N. Satterfield, 2^{nd} ed. (1991), Seite 8). Nach diesem Konzept wurde die Umsatzrate als turnover frequency (TON) auf ein katalytisches Zentrum bezogen (siehe "Heterogeneous Catalysis in Industrial practice", Charles N. Satterfield, 2^{nd} ed. (1991), Seite 15). Danach würde der Fachmann bei Anwesenheit von zwei Katalysatoren oder katalytischen Zentren erwarten, dass sich die Umsatzraten dieser beiden addieren.

Es ist auch die sogenannte heterohomogene Katalyse bekannt (siehe beispielsweise "Heterogeneous Catalysis in Industrial practice", Charles N. Satterfield, 2^{nd} ed. (1991), Seite 13/14), wonach ein heterogener Katalysator beispielsweise Radikale bildet und diese Radikale im flüssigen Reaktionsmedium eine Kettenreaktion auslösen. Bei heterohomogenen Katalysen kann auch die katalytische Reaktion am heterogenen Katalysator mit einer homogenen Reaktion in Wechselwirkung treten. Es ist aber nicht bekannt und wird auch durch dieses Dokument nicht nahegelegt, dass eine bereits homogen katalysierte Reaktion durch anwesende poröse Feststoffpartikel, die ihrerseits als Katalysaor für die betrachtete Reaktion unter den Reaktionsbedingungen allein nicht oder nur mäßig katalytisch wirksam sind, in ähnlicher Weise wie im Falle der Beschleunigung der thermischen Zersetzung von Gasen durch Zugabe eines weiteren inerten Gases (siehe "Heterogeneous Catalysis in Industrial practice", Charles N. Satterfield, 2^{nd} ed. (1991), Seite 9), weiter beschleunigt werden kann.

Aufgabe der Erfindung ist demgemäß die Bereitstellung eines Verfahrens zur Durchführung einer homogen katalysierten Umsetzung eines Edukts unter Bildung eines Reaktionsprodukts, wobei die Wirkung des homogenen Katalysators verstärkt und damit die Umsetzung weiter beschleunigt wird.

Dass es sich bei dem erfindungsgemäßen Effekt um eine verstärkende Wirkung des Katalysators und nicht um einen zusätzlichen Katalysator nach obiger Definition handelt, ist daran zu erkennen, dass die Umsatzrate nicht additiv um die Umsatzrate eines zusätzlichen Katalysators, also der porösen Feststoffpartikel, erhöht wird, sondern eher multiplikativ um einen bestimmten Faktor erhöht beziehungsweise verstärkt wird.

Die Aufgabe lässt sich überraschenderweise dadurch lösen, dass die Umsetzung in Gegenwart von porösen Feststoffpartikeln, insbesondere eines Festbetts aus solchen porösen Partikeln durchgeführt wird. Offensichtlich wirken die porösen Feststoffpartikel primär nicht durch eingebaute oder adsorbierte aktive Zentren, wie Säure oder Metallzentren, sondern allein durch die dreidimensionale Struktur der porösen Partikel. Eine Wechselwirkung zwischen der dreidimensionalen Struktur und/oder Aktivitätszentren der porösen Partikel mit dem homogenen Katalysator wird nicht ausgeschlossen.

Gegenstand der Erfindung ist demgemäß ein Verfahren zur Durchführung einer homogen katalysierten Umsetzung, wobei ein Edukt unter Abspaltung oder Anlagerung eines Reaktionspartners oder unter Isomerisierung in flüssiger Phase, welche einen für die Umsetzung geeigneten Katalysator in wirksamer Menge in gelöster Form enthält, in ein Reaktionsprodukt überführt wird, das dadurch gekennzeichnet ist, dass man die das Edukt und den homogenen Katalysator enthaltende flüssige Phase sowie, soweit erforderlich, anzulagernde Reaktionspartner mit einem stationären oder bewegten Bett aus porösen Partikeln in Kontakt bringt, insbesondere durch ein stationär angeordnetes Festbett leitet. Die Unteransprüche richten sich auf bevorzugte Ausführungsformen.

Das homogen katalysierte flüssige Reaktionsmedium sowie gegebenenfalls an ein Edukt anzulagernde Reaktionspartner können in beliebiger Weise in diskontinuierlichen oder kontinuierlichen Prozessen mit den porösen Feststoffpartikeln in Kontakt gebracht werden. Möglich ist es damit, das flüssige Reaktionsmedium über einem stationären oder bewegten Bett aus porösen Feststoffpartikeln stehen zu lassen, das flüssige Reaktionsmedium über dieses Festbett zu leiten oder die porösen Feststoffpartikel analog einem Suspensionskatalysator im flüssigen Reaktionsmedium in Suspension zu halten. Gemäß einer bevorzugten Ausführungsform werden die porösen Feststoffpartikel in einem Reaktor als Festbett angeordnet und das flüssige Reaktionsmedium in geflutetem Zustand (= Blasen-Fahrweise) oder durch Berieseln (= Rieselbett-Fahrweise) über das Festbett geleitet. Durch das erfindungsgemäße Verfahren wird die Reaktionsgeschwindigkeit erheblich gesteigert.

Die Rieselbett-Fahrweise wird besonders bevorzugt. Da der Flüssigkeits-hold up in einem Rieselbettreaktor im allgemeinen 5 bis 20 % des Festbettvolumens beträgt (siehe "Multiphase Chemical Reactors" ed. A. Gianetto and P.L. Silveston, Hemisphere Publ. Corp. (1986), Seite 9), kann, wie sich aus den erfindungsgemäßen Beispielen ergibt, die homogen katalysierte Reaktion um mehr als den Faktor 10 beschleunigt werden. Die Rieselbett-Fahrweise eignet sich insbesondere in solchen Systemen, in welchen außer dem flüssigen Reaktionsmedium ein gasförmiger Reaktionspartner, wie Wasserstoff im Falle einer Hydrierung, teilnimmt.

Erfindungswesentliches Merkmal außer der Anwesenheit poröser Feststoffpartikel ist, dass die Umsetzung durch einen im flüssigen Reaktionsmedium gelösten homogenen Katalysator katalysiert wird. Das erfindungsgemäße Verfahren ist von der Art des im flüssigen Reaktionsmedium gelösten Katalysators und der katalysierten Umsetzung im wesentlichen unabhängig.

Es wurde festgestellt, dass säurekatalysierte Umsetzungen in wässrigen Systemen auch in Abwesenheit eines dem Reaktionsmedium zugesetzten Säurekatalysators an porösen Feststoffen erheblich beschleunigt werden können. In diesem Fall fungiert das Wasser als homogener saurer Katalysator.

Bei der Umsetzung kann es sich beispielsweise um eine säurekatalysierte Umsetzung, wie z. B. eine Veretherung, Etherspaltung, Veresterung, Esterspaltung, Umesterung, Acetalisierung, Ketalisierung, Iminierung, Hydrolyse von Iminen, Nitrilen und Anhydriden, Dehydratisierung von Alkoholen und Isomerisierung von olefinischen Verbindungen handeln. Bei der säurekatalysierten Umsetzung enthält das Reaktionsmedium eine wirksame Menge einer organischen oder anorganischen Säure, insbesondere eine niedere Carbonsäure oder Mineralsäure, in gelöster Form. Auch die Protonen des Wassers allein können als Säure fungieren. Die Auswahl und Menge des sauren Katalysators richten sich nach dem betrachteten System und den gewünschten Reaktionsbedingungen und sind Gegenstand fachmännischer Optimierung.

Homogen durch Basen katalysierte Reaktionen, wie beispielsweise die Hydrolyse von Amiden oder die Anlagerung von Cyanwasserstoff Aldehyde, Ketone und aktivierte Olefine, lassen sich durch das erfindungsgemäße Verfahren unter Einsatz von z.B. Alkalihydroxiden als Katalysator weiter beschleunigen.

Auch homogen katalysierte Hydrierungen sind dem erfindungsgemäßen Verfahren zugänglich. Homogene Katalysatoren sind hierbei beispielsweise im Reaktionsmedium lösliche Edelmemallverbindungen und Verbindungen von Eisen, Cobalt und Nickel.

Weitere dem Verfahren zugängliche homogen katalysierte Reaktionen sind Dehydrierungs- und Oxidationsreaktionen, zum Beispiel solche unter Einsatz von löslichen Verbindungen von Elementen aus der Reihe Chrom, Vanadium, Molybdän und Wolfram und Hydroformulierungen, zum Beispiel solche in Gegenwart ligandenstabilisierter Co-Carbonylverbindungen.

Die porösen Feststoffpartikel führen, unabhängig davon, ob sie selbst die gewünschte Reaktion gar nicht oder mehr oder weniger stark katalysieren, zu einer signifikanten Steigerung der Beschleunigung der Reaktion; bei einer Eigenkatalyse durch die Feststoffpartikel wird eine synergistische Wirkung der Beschleunigung der homogen katalysierten Reaktion erzielt.

Gemäß einer weiteren Ausführungsform der Erfindung werden als poröse Feststoffpartikel solche Stoffe verwendet, welche einerseits durch ihre dreidimensionale Struktur eine homogen katalysierte erste Reaktion weiter beschleunigen, andererseits aber auch Aktivitätszentren aufweisen, die eien zweite, parallel oder nachgeschaltet ablaufende Reaktion heterogen katalysieren. Diese Ausführungsform ist besonders bei der säurekatalysierten Reaktion zweckmäßig, wenn während der homogen katalysierten ersten Reaktion als Nebenprodukt gebildete ungesättigte Verbindungen sofort hydriert werden sollen - in diesem Fall enthalten die Feststoffpartikel hydrierwirksame Aktivitätszentren aus Edelmetallen und/oder Nickel oder Cobalt, und die als zweite Reaktion ablaufende Hydrierung wird heterogen katalysiert.

Die erfindungsgemäß zu verwendenden porösen Feststoffpartikel enthalten Poren aus der Reihe von Makroporen mit einem Porendurchmesser von größer 50 nm (50 bis 10.000 nm), Mesoporen mit einem Porendurchmesser im Bereich von 2 bis 50 nm und Mikroporen mit einem Porendurchmesser von kleiner 2 nm. Besonders vorteilhaft sind Feststoffe mit einem hohen Anteil an Meso- und Mikroporen. Das Porenvolumen der Feststoffe liegt zweckmäßigerweise im Bereich von 0,1 bis 3 ml/g, vorzugsweise im Bereich von 0,3 bis 1,5 ml/g. Gemäß einer bevorzugten Ausführungsform bestehen die Poren zu 10 bis 100 % aus Mikro- und Mesoporen, wobei besonders bevorzugt 40 bis 80 % des Porenvolumens aus Mesoporen gebildet wird. Das Mikroporenvolumen lässt sich aus der Stickstoff-Adsorptionsisotherme bei der Temperatur des flüssigen Stickstoffs durch Vergleich mit einer Standardisotherme nach dem t-plot-Verfahren von De Boer (vgl. De Boer et al. in J. of Colloid an Interface Science 21, 405-44 (1966)) nach DIN 66135, Teil 2 (Entwurf - April 1988) bestimmen. Die Bestimmung des Mesoporenvolumens und der Porenverteilung kann aus der Stickstoff-Desorptionsisotherme nach Barett, Joyner und Halenda gemäß DIN 66134 (Februar 1998) erfolgen. Die zur Bestimmung des Mikro- und Mesoporenvolumens eingesetzte Probe wird vor der Messung 1 h bei 200 °C im Vakuum (kleiner 1,3 Pa) behandelt. Die Messung erfolgt beispielsweise in einem Gerät "ASAP 2400" der Firma Micromeritics, Norcross, Ga. (US). Die Makroporen werden durch Quecksilber-Intrusion nach DIN 66133 oder ASTM D 4284 bestimmt.

Das Material der porösen Feststoffpartikel ist üblicherweise gegenüber der betrachteten Umsetzung und den Einsatzstoffen im wesentlichen inert. Beispielsweise handelt es sich um Aktivkohle, oxidische, silikatische oder metallische Materialien oder um poröse Mehrkomponentensysteme. Besonders bevorzugt und im Handel in unterschiedlicher Porosität erhältlich sind Aktivkohlen, oxidische Stoffe, wie TiO₂, Al₂O₃ und SiO₂, und silikatische Stoffe, wie Alsilikate, Zeolithe und Titansilikalite. Der Fachmann wird die Stoffauswahl außer von der Porosität auch davon abhängig machen, ob dem Feststoff bei der betrachteten Umsetzung eine zusätzliche Funktion zukommt. Bei Bedarf wird der Stoff in dieser Hinsicht nachbehandelt, beispielsweise durch Dotieren mit heterogen-katalytisch wirksamen Elementen oder Verbindungen, wie einem Platinmetall zur Erzeugung einer Hydrieraktivität.

Außer der Kontaktzeit des flüssigen Reaktionsmediums am porösen Feststoff sind die Reaktionsbedingungen, darunter das Molverhältnis der Edukte, Reaktionspartner, Lösungsmittel, Temperatur und Druck von der gewünschten Umsetzung abhängig und dem Fachmann aus der einschlägigen Fachliteratur leicht zugänglich. Die Kontaktzeit lässt sich leicht durch Optimierung ermitteln.

Wie aus den nachfolgend gezeigten Beispielen und Vergleichsbeispielen folgt, wird durch den erfindungsgemäßen Einsatz eines Festbetts aus porösen Feststoffen die Umsetzung signifikant beschleunigt. Gleichzeitig wird durch die Beschleunigung der Zielreaktion vielfach die Selektivität erhöht.

Die Vorteile der Erfindung liegen in ihrer breiten Anwendbarkeit, der Steigerung der Beschleunigung der homogen katalysierten Umsetzung, der Möglichkeit eine höhere Selektivität zu erzielen sowie der Kombinationsfähigkeit mit einer heterogenen katalysierten Umsetzung.

### Beispiele

### Vergleichsbeispel 1 (VB1)

Säurekatalysierte Dehydratisierung von Sorbitol:
In einem 2 l-Autoklaven wurden 1,5 kg einer 20 gew.-%igen wässrigen D-Sorbitollösung sowie 15 g Propionsäure (= 5 Gew.-%, bezogen auf D-Sorbitol) als Homogenkatalysator vorgelegt; zugegeben wurden 3 g eines Pd-Aktivkohle-Katalysators (Pd-Gehalt 3 Gew.-%). Das Reaktionsgemisch wurde auf 250 °C erwärmt und 1,5 h bei 8 MPa Wasserstoffdruck gerührt. Nach dem Abkühlen wurde der Hydrierkatalysator abfiltriert. Der D-Sorbitolumsatz betrug 71 %. Bezogen auf das Reaktorvolumen und die Reaktionszeit betrug der Umsatz 94,7 g/l·h. Die Selektivität an Dianhydrosorbitol betrug 8 %, bezogen auf den Sorbitolumsatz.

### Vergleichsbeispiel 2 (VB2)

VB1 wurde ohne die Anwesenheit von Propionsäure wiederholt. Das schwach saure Sorbitol selbst fungierte als Homogenkatalysator. Der D-Sorbitolumsatz betrug 46 % bzw. 61,2 g/l·h. Die Selektivität an Dianhydrosorbitol betrug 4 %, bezogen auf den Sorbitolumsatz.

### Vergleichsbeispiel 3 (VB3)

VB1 wurde mit dem Unterschied wiederholt, dass der Hydrierkatalysator weggelassen wurde. Der D-Sorbitolumsatz betrug 73 %, entsprechend 97,3 g/l·h. Neben Anhydrosorbitol entstanden jedoch 10 % eines gelben Polymers.

### Vergleichsbeispiel 4 (VB4)

VB1 wurde mit dem Unterschied wiederholt, dass die Reaktionszeit 3,5 h betrug. Der D-Sorbitolumsatz betrug 95 %, entsprechend 54,3 g/l·h. Die Selektivität an Dianhydrosorbitol betrug 17 %, bezogen auf den Sorbitolumsatz.

### Vergleichsbeispiel 5 (VB5)

VB2 wurde mit dem Unterschied wiederholt, dass die Reaktionszeit 3,5 h betrug. Der D-Sorbitolumsatz betrug 78 %, entsprechend 44,5 g/l·h. Die Selektivität an Dianhydrosorbitol betrug 11 %, bezogen auf den Sorbitolumsatz.

### Beispiel 1 (B1)

In einem Reaktionsrohr mit 18 mm Innendurchmesser und 91 ml effektivem Volumen wurden 70 ml Aktivkohle Strangpreßlinge vorgelegt. Die Aktivkohle hatte eine spezifische Oberfläche von 1530 m²/g und folgende Porenvolumina: Mikroporen 0,68 ml/g, Mesoporen 0,26 ml/g, Makroporen 0,36 ml/g. Die Aktivkohle war mit 0,05 Gew.-% Palladium dotiert. 70 ml einer solchen Aktivkohleschüttung wurden in den Reaktor eingefüllt. Eine wässrige Lösung, enthaltend 20 Gew.-% D-Sorbitol und 5 Gew.-% Propionsäure-Katalysator, wurde über Kopf zusammen mit dem Wasserstoffgas aufgegeben. Der Druck betrug 80 MPa, der Wasserstofffluß 20 Nl/h. Der Flüssigkeitsfluß betrug 47 ml/h. Das Reaktionsrohr wurde mit Hilfe eines Doppelmantels beheizt, die Reaktorinnentemperatur betrug 250 °C. Die Reaktionsmischung wurde gaschromatografisch analysiert. Es ergab sich ein D-Sorbitolumsatz von 96 %, entsprechend einem Umsatz bezogen auf das Festbettvolumen und die Zeit von 129 g/l·h. Die Selektivität an Dianhydrosorbitol betrug 31 %, bezogen auf den Sorbitolumsatz.

Bei einem literaturgemäßen Flüssigkeitsanteil im Rieselbettreaktor von 5 bis 20 % ermittelt sich bei dem angegebenen Fluss von 47 ml/h eine Verweilzeit der Flüssigkeit am Festbett und damit eine Reaktionsdauer im Bereich von 0,075 bis 0,3 h. Im Vergleichsbeispiel 1 betrug demgegenüber die Reaktionsdauer 1,5 h, und zudem wurden ein geringerer Umsatz und niedrigere Selektivität erzielt. Wie VB4 zeigt, ist ohne den Zusatz eines porösen Festkörpers eine Steigerung der Reaktionsdauer um den Faktor 2,3 notwendig, um etwa den gleichen Umsatz zu erzielen. Dieser Vergleich zeigt den erfindungsgemäß erzielten überraschenden Effekt.

### Beispiel 2 (B2)

B1 wurde wiederholt, jedoch enthielt das Reaktionsgemisch keine Propionsäure. Das schwach saure Sorbitol selbst fungierte als Homogenkatalysator. Der D-Sorbitolumsatz betrug 76 % bzw. 102 g/l·h (g Sorbitol pro l Festbett und Stunde). Die Selektivität an Dianhydrosorbitol betrug 19 %, bezogen auf den Sorbitolumsatz. Wie VB5 zeigt, ist auch hier ohne den Zusatz eines porösen Festkörpers eine Steigerung der Reaktionsdauer um den Faktor 2,3 notwendig, um etwa den gleichen Umsatz zu erzielen.

## Patentansprüche

1. Verfahren zur Durchführung einer homogen katalysierten Umsetzung, wobei ein Edukt unter Abspaltung oder Anlagerung eines Reaktionspartners oder unter Isomerisierung in flüssiger Phase, welche einen für die Umsetzung geeigneten Katalysator in wirksamer Menge in gelöster Form enthält, in ein Reaktionsprodukt überführt wird,
dadurch gekennzeichnet,
dass man die das Edukt und den homogenen Katalysator enthaltende flüssige Phase sowie, soweit erforderlich, anzulagernde Reaktionspartner mit einem stationären oder bewegten Bett aus porösen Partikeln in Kontakt bringt.

2. Verfahren nach Anspruch 1,
dadurch gekennzeichnet,
dass man die flüssige Phase in Rieselbett-Fahrweise über ein stationäres Festbett leitet.

3. Verfahren nach Anspruch 1 oder 2,
dadurch gekennzeichnet,
dass man eine säurekatalysierte Umsetzung aus der Reihe Veretherung, Etherspaltung, Veresterung, Esterspaltung, Umesterung, Acetalisierung, Iminierung, Dehydratisierung von Alkoholen und Hydrolyse von Acetalen, Iminen und Nitrilen unter Einsatz einer in der flüssigen Phase gelösten anorganischen oder organischen Säure als Katalysator durchführt.

4. Verfahren nach Anspruch 1 oder 2,
dadurch gekennzeichnet,
dass man eine homogen katalysierte Hydrierung durchführt, wobei eine einen homogenen Hydrierkatalysator und ein Edukt enthaltende flüssige Phase in Gegenwart von Wasserstoff über ein Festbett aus porösen Partikeln geleitet wird.

5. Verfahren nach einem der Ansprüche 1 bis 4,
dadurch gekennzeichnet,
dass man ein Festbett aus Aktivkohlepartikeln oder oxidischen oder silikatischen Partikeln verwendet.

6. Verfahren nach einem der Ansprüche 1 bis 5,
dadurch gekennzeichnet,
dass man Festbett-Partikel mit einem Porenvolumen im Bereich von 0,1 bis 3 ml/g verwendet.

7. Verfahren nach Anspruch 6,
dadurch gekennzeichnet,
dass 10 bis 100 % des Porenvolumens aus Mikro- und Mesoporen gebildet wird.
